Europäisches Patentamt

European Patent Office

Office européen des brevets

(11) Veröffentlichungsnummer: **0 341 197**
**A1**

## (12) EUROPÄISCHE PATENTANMELDUNG

(21) Anmeldenummer: **89730115.6**

(22) Anmeldetag: **02.05.89**

(51) Int. Cl.⁴: **A 61 B 17/58**

(30) Priorität: **30.04.88 DE 8805887**

(43) Veröffentlichungstag der Anmeldung:
**08.11.89 Patentblatt 89/45**

(84) Benannte Vertragsstaaten:
**AT BE CH DE ES FR GB GR IT LI NL SE**

(71) Anmelder: **MECRON medizinische Produkte GmbH**
**Nunsdorfer Ring 23-29**
**D-1000 Berlin 48 (DE)**

(72) Erfinder: **Wicki, Otto, Dr.**
**Kantonales Spital Wolhusen**
**CH-6110 Wolhusen (CH)**

(74) Vertreter: **Christiansen, Henning, Dipl.-Ing.**
**Patentanwalt CHRISTIANSEN Pacelliallee 43/45**
**D-1000 Berlin 33 (DE)**

(54) **Haltevorrichtung für orthopädische Zwecke.**

(57) Haltevorrichtung für orthopädische Zwecke, wobei zwei einarmige Hebel um eine vertikale Achse (4) drehbar gelagert sind, wobei die Hebelenden als Griffe (5, 6) ausgebildet sind, die Innenseiten mindestens eines Hebels jeweils mindestens einen Bereich mit konkaver Ausformung (7 bis 10) zwischen Griff und Drehachse aufweisen, der mit einer Profilierung zur Verbesserung des Reibschlusses versehen ist und die Drehachse mit einer eine Standfläche bildenden Grundplatte (11) fest verbunden ist.

Bundesdruckerei Berlin

## Beschreibung

### Haltevorrichtung für orthopädische Zwecke

Die Erfindung betrifft eine Haltevorrichtung der im Oberbegriff des Anspruchs 1 angegebenen Art.

Bei orthopädischen Operationen besteht das Bedürfnis nach einer Spannvorrichtung, welche es ermöglicht, den Femur kopf zur Bearbeitung schnell und sicher festzulegen, um ihn spanabhebend zu bearbeiten.

Bekannte Spannvorrichtungen sind für diesen Zweck nicht geeignet, weil sie nicht den Arbeitsverhältnissen während einer orthopädischen Operation angepaßt sind.

Der Erfindung liegt die Aufgabe zugrunde, bei eine Haltevorrichtung der eingangs genannten Gattung eine einfache Handhabung zu ermöglichen, wobei die Vorrichtung auch noch konstruktiv einfach ausgebildet sein muß.

Diese Aufgabe wird mit den kennzeichnenden Merkmalen des Anspruchs 1 gelöst.

Die Erfindung beruht auf der Erkenntnis, daß für die genannte Aufgabenstellung eine Vorrichtung geschaffen werden muß, welche einerseits mit nur einem Handgriff einen sicheren Halt für den zu bearbeitenden Femurkopf gibt und andererseits auch einen sicheren Halt auf einer Standfläche bietet.

Besonders vorteilhaft dabei ist, wenn einer der Hebel fest mit der Standfläche verbunden ist da die Vorrichtung damit standlicher auf einer Unterlage abstellbar ist. Eine Spannschraube, welche die Öffnungsweite der Hebel begrenzt, ermöglicht eine schnelle Arretierung, insbesondere wenn sie sich an der Außenseite des einen Hebels abstützt und mit einer Gewindestange in Eingriff ist, welche mit dem anderen Hebel verbunden ist und eine durchgehende Aussparung des ersten Hebels durchquert. Die Gewindestange ist dabei bevorzugt um eine zur Drehachse der Hebel parallele Achse schwenkbar gelagert.

Zur Erhöhung der Haltewirkung der Backen sind die prismatisch ausgebilden, spiegelsymmetrischen Ausnehmungen mit einer Profilierung versehen. Eine Profilierung, die aus pyramidenförmigen Körpern besteht, verhindert nicht nur ein ungewolltes Verdrehen, sondern auch ein Verschieben der zu bearbeitenden Knocheteile in einer Richtung parallel zur Schwenkachse der Hebel.

Bevorzugt sind mehrere mit Profilierung versehene konkave Bereiche unterschiedlicher Krümmung aneinandergrenzend vorgesehen, so daß auch Knochenteile unterschiedlicher Größe gefaßt und bearbeitet werden können.

Wenn die Grundplatte eine Ein- oder Auflage aus Kunststoff aufweist, die insbesondere auswechselbar und körperverträglich ausgebildet ist.

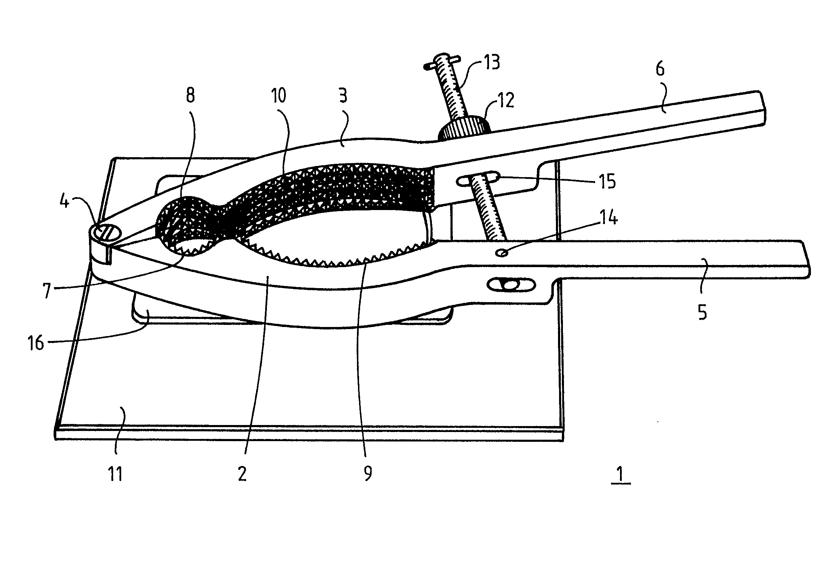

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figur näher dargestellt. Die einzige Figur zeigt das Ausführungsbeispiel in perspektivischer Darstellung.

Bei dem in der Figur dargestellten Ausführungsbeispiel einer Haltevorrichtung 1 für orthopädische Zwecke sind zwei einarmige Hebel 2 und 3 um eine vertikale, durch eine Schraube 4 gebildete Achse relativ zu einander drehbar ge lagert. Die freien Hebelenden sind als Griffe 5 und 6 ausgebildet. Die beiden Griffteile sind im wesentlichen spiegelbildlich gestaltet.

Die Innenseiten der beiden Hebel sind jeweils nahe der Drehachse mit konkaven Ausformungen versehen, die in einer Richtung parallel zur Drehachse im wesentlichen prismatisch ausgebildet sind. Und zwar sind nahe der Drehachse konkave Ausnehmungen 7 und 8 mit relativ großer Krümmung - aber kleinerer Öffnungsweite - vorgesehen. An diese schließen sich entsprechende Ausnehmungen 9 und 10 kleinerer Krümmung - aber größerer Öffnungsweite an. Die größere Öffnungsweite ist auf Femurköpfe abgestimmt, während mit den kleineren Öffnungsweite sonstige kleinere Knochenteile gehalten werden können. Bei anderen vorteilhaften - in der Zeichnung nicht dargestellten - Ausführungen ist auch ein kontinuierlicher diskontinuierlicher Übergang zwischen den Öffnungsweiten in sonstiger Weise möglich. Die Innenflächen der Ausnehmungen 7 bis 10 sind mit Profilierungen zur Verbesserung des Reibschlusses versehen. Diese sind bevorzugt als - insbesondere vierseitige - pyramidenförmige Erhungen ausgebildet und bestehen aus dem Metallwerkstoff der Vorrichtung - insbesondere rostfreiem Stahl.

Der Hebel 2 ist mit einer eine Standfläche bildenden Grundplatte 11 fest verbunden. Auf diese Weise ist die Vorrichtung standfest und kippsicher. Zur schnellen Arretierung ist eine als Rändelschraube 12 ausgebildete Spannschraube vorgesehen, welche einen Anschlag für den beweglichen Hebelarm 3 bildet und die Öffnungsweite der Hebel begrenzt.

Die Spannschraube stützt sich dabei an der Außenseite des bewegliche Hebels ab und ist mit einer Gewindestange 13 in Eingriff, welche in dem fest mit der Grundplatte 11 verbundenen Hebel um eine durch einen Stift 14 gebildete Achse schwenkbar gelagert ist. Die Gewindestange durchquert eine parallel zu Grundplatte 11 gerichtete durchgehende Aussparung 15 des Hebels 3.

Die Grundplatte 11 weist im Schwenkbereich der Hebel unterhalb der Ausnehmungen 7 bis 10 eine wenige Millimeter starke Platte 16 aus Kunststoff, insbesondere aus Polyethylen oder einem anderen körperverträglichen Werkstoff, auf welcher als Arbeitsfläche bei der Knochenbarbeitung dient. Durch die körperverträgliche Werkstoffwahl ist es unproblematisch, wenn mit den bei der Bearbeitung erzeugten Knochenspänen Kunststoffspäne mit zur Implantation gelangen.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

**Patentansprüche**

1. Haltevorrichtung für orthopädische Zwecke,
**dadurch gekennzeichnet,**
daß zwei einarmige Hebel um eine vertikale Achse (4) drehbar gelagert sind, wobei
die Hebelenden als Griffe (5, 6) ausgebildet sind,
die Innenseiten mindestens einen Hebels jeweils mindestens einen Bereich mit konkaver Ausformung (7 bis 10) zwischen Griff und Drehachse aufweisen, der mit einer Profilierung zur Verbesserung des Reibschlusses versehen ist und
die Drehachse mit einer eine Standfläche bildenden Grundplatte (11) fest verbunden ist.

2. Haltevorrichtung nach Anspruch 1, **dadurch gekennzeichnet,** daß einer der Hebel (2) fest mit der Grundplatte (11) verbunden ist.

3. Haltevorrichtung nach einem der Ansprüche 1 oder 2, **dadurch gekennzeichnet,** daß eine Spannschraube (12), insbesondere Rändelschraube, vorgesehen ist, welche die Öffnungsweite der Hebel (2, 3) begrenzt.

4. Haltevorrichtung nach einem der vorangehenden Ansprüche, **dadurch gekennzeichnet,** daß die Spannschraube (12) sich an der Außenseite des einen Hebels (3) abstützt und mit einer Gewindestange (13) in Eingriff ist, welche mit dem anderen Hebel (2) verbunden ist und eine durchgehende Aussparung (15) des ersten Hebels (3) durchquert.

5. Haltevorrichtung nach Anspruch 4, **dadurch gekennzeichnet,** daß die Gewindestange mit dem ersten Habel um eine zur Drehachse der Hebel parallele Achse (14) schwenkbar gelagert ist.

6. Haltevorrichtung nach einem der vorangehenden Ansprüche, **dadurch gekennzeichnet,** daß die mit Profilierung versehenen konkaven Bereich (7 bis 10) in Richtung der Drehachse prismatisch ausgebildet ist.

7. Haltevorrichtung nach einem der vorangehenden Ansprüche, **dadurch gekennzeichnet,** daß mit Profilierung versehene konkave Bereiche (7 bis 10) der beiden Hebel spiegelsymmetrisch angeordnet sind.

8. Haltevorrichtung nach einem der vorangehenden Ansprüche, **dadurch gekennzeichnet,** daß mehrere mit Profilierung versehene konkave Bereiche (7 bis 10) unterschiedlicher Krümmung aneinandergrenzen.

9. Haltevorrichtung nach einem der vorangehenden Ansprüche, **dadurch gekennzeichnet,** daß die Grundplatte (11) eine Ein- oder Auflage (16) aus Kunststoff aufweist.

10. Haltevorrichtung nach Anspruch 9, **dadurch gekennzeichnet,** daß der Kunststoff körperverträglich - insbesondere Polyethylen - ist.

EP 0 341 197 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CH-A- 607 915 (SYNTHES) <br> * Seite 2, Spalte 2, Zeilen 35-41; Seite 3, Spalte 1, Zeile 6 - Spalte 2, Zeile 3; Figur 1 * <br> --- | 1,3-8 | A 61 B 17/58 |
| A | US-A-2 786 272 (LINDLEY) <br> * Spalte 2, Zeilen 59-63; Spalte 2, Zeile 71 - Spalte 3, Zeile 4; Figur 1 * <br> ----- | 1,9,10 | |

|  |  |  | RECHERCHIERTE SACHGEBIETE (Int. Cl.4) |
|---|---|---|---|
|  |  |  | A 61 B <br> A 61 C <br> A 61 F |

Der vorliegende Recherchenbericht wurde für alle Patentansprüche erstellt

| Recherchenort | Abschlußdatum der Recherche | Prüfer |
|---|---|---|
| DEN HAAG | 17-07-1989 | MOERS R.J. |